(19) Europäisches Patentamt
European Patent Office
Office européen des brevets

(11) EP 2 456 775 B1

(12) **EUROPEAN PATENT SPECIFICATION**

(45) Date of publication and mention of the grant of the patent:
**21.08.2013 Bulletin 2013/34**

(21) Application number: **10734121.6**

(22) Date of filing: **19.07.2010**

(51) Int Cl.:
***C07D 501/46*** *(2006.01)* ***A61K 31/545*** *(2006.01)*

(86) International application number:
**PCT/EP2010/060376**

(87) International publication number:
**WO 2011/009827 (27.01.2011 Gazette 2011/04)**

# (54) METHOD OF MAKING CEFQUINOME PARTICLES

Verfahren zur Herstellung von Cefquinom-Teilchen

Procédé de fabrication de particules de cefquinome

(84) Designated Contracting States:
**AL AT BE BG CH CY CZ DE DK EE ES FI FR GB GR HR HU IE IS IT LI LT LU LV MC MK MT NL NO PL PT RO SE SI SK SM TR**

(30) Priority: **20.07.2009 EP 09165895**
**21.07.2009 US 227195 P**

(43) Date of publication of application:
**30.05.2012 Bulletin 2012/22**

(73) Proprietor: **Intervet International B.V.**
**5831 AN Boxmeer (NL)**

(72) Inventors:
- **NIEDERMANN, Hans, Peter**
**55270 Schwabenheim (DE)**
- **BOTHE, Heiko**
**55270 Schwabenheim (DE)**

(74) Representative: **Stumm, Karin**
**Merck Sharp & Dohme Ltd.**
**Hertford Road**
**Hoddesdon, Hertfordshire**
**EN11 9BU (GB)**

(56) References cited:
**EP-A- 0 280 157** **WO-A-03/063877**
**WO-A-2007/145656** **CN-A- 101 536 985**
**US-A1- 2004 115 260** **US-A1- 2006 100 424**

- **EHINGER ET AL: "Tissue distribution of cefquinome after intramammary and ''systemic'' administration in the isolated perfused bovine udder" 1 July 2006 (2006-07-01), VETERINARY JOURNAL, BAILLIERE TINDALL, LONDON, GB, PAGE(S) 147 - 153 , XP005492215 ISSN: 1090-0233 the whole document**
- **DATABASE WPI Week 200968 Thomson Scientific, London, GB; AN 2009-P17793 XP002603541 & CN 101 536 985 A (TIANJIN RINGPU BIOLOGICAL TECHNOLOGY GROUP) 23 September 2009 (2009-09-23)**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 456 775 B1

## Description

### Field of the Invention

The invention pertains to the production of particles of a cefquinome acid addition salt. Particularly, the invention pertains to a method of making cefquinome sulfate crystals of a desired particle size range, without high energy particle size reduction such as milling or micronisation..

### Background of the Invention

Cefquinome is an antibiotic used for humans and animals, *inter alia,* to treat bovine respiratory disease, *Pasteurella* infections in pigs, and many other applications where a high antibacterial activity is desired. Cefquinome generally comes as an acid addition salt, preferably a sulfate. The cefquinome sulfate has a chemical structure of the following formula:

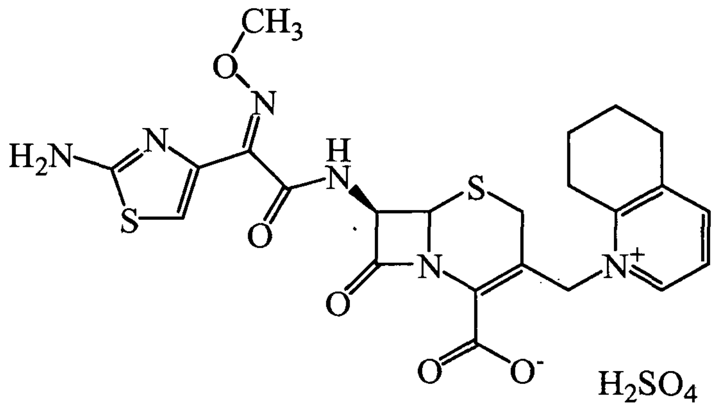

The chemical name for this structure is 1-[[6R,7R)-7-[2-(2-amino-4-thiazolyl)glyoxylamido]-2-carboxy-8-oxo-5-thia-1-azabicyclo[4.2.0]oct-2-en-3-yl]methyl]-5,6,7,8-tetrahydroquinolinium hydroxide, inner salt $7^2$-(Z)-(O-methyloxime), sulfate.

The synthesis of cefquinome sulfate and other acid addition salts is known, e.g., from EP 280 157 or US 2006/0100424.

WO03/063877 discloses cefquinome compositions for intra-mammary administration in cattle.

Existing process for making sterile cefquinome sulfate particles comprise the steps of providing a suspension of cefquinome sulfate in water; adding alkaline (typically NaOH) to the sulfate, followed by an organic solvent, (typically acetone), resulting in the precipitation of the corresponding sulfate salt ($Na_2SO_4$), and the retaining of a solution of cefquinome free base (which in fact is a betaine); conducting sterile filtration of the betaine solution; and adding sulfuric acid so as to produce sterile cefquinome sulfate particles or adding any other acids to produce any corresponding sterile cefquinome salt particles.

The drug is presented *inter alia* as an injection preparation, typically in the form of suspensions, e.g. in ethyl oleate or in the type of esters of saturated coconut and palm kernel oil-derived caprylic and capric fatty acids and glycerine or propylene glycol, known by the trade name of Miglyol. As described in WO 03/063877 the use of an antibacterial agent in the form of small particles of a narrow particle size range is advantageous compared to coarser material. A practically feasible size distribution of such small particles is, e.g.,

$$d(50) \leq 7\ \mu m$$

$$d(90) \leq 15\ \mu m$$

$$d(100) \leq 50\ \mu m$$

Hereto the aforementioned sterile cefquinome sulphate particles, prepared according to EP 280157 or US

2006/0100424, are too coarse and have to be reduced in size, by high energy particle size reduction methods, typically by milling or micronizing.

Although cefquinome generally yields stable products, a desire exists to even further improve cefquinome particles in terms of physical stability and to provide cefquinome suspensions that are stable, particularly with reference to lower vulnerability for sedimentation. Moreover, the foregoing should preferably be realized on the basis of cefquinome particles that satisfy the aforementioned size distribution.

**Summary of the Invention**

In order to better address one or more of the foregoing desires, the invention, in one aspect, presents a process for the production of particles of a cefquinome acid addition salt, preferably cefquinome sulfate, by precipitation of the cefquinome acid addition salt from a cefquinome betaine solution, wherein acid is added to the betaine solution, and wherein the addition of the acid, particularly sulfuric acid, is done in a single shot in a molar excess of 40% to < 100%.

The invention, in another aspect, presents cefquinome acid addition salt particles, particularly cefquinome sulfate particles, obtainable by the foregoing method.

In a still further aspect, the invention presents crystals of cefquinome acid addition salt, particularly cefquinome sulfate, preferably in the range from 0.05 $\mu$m to 100 $\mu$m.

In yet another aspect, the invention provides particles of a cefquinome acid addition salt, particularly cefquinome sulfate particles, of a size distribution $d(50) \leq 7\mu$ m, $d(90) \leq 15$ $\mu$m, and $d(100) \leq 50$ $\mu$m, wherein the particles are not milled or micronized.

**Detailed Description of the Invention**

In a first aspect, the invention is a process for the production of particles of a cefquinome acid addition salt. With reference to the above-described background, the process of the invention is based on a solution of cefquinome free base (i.e. a betaine solution).

In order to prepare an acid addition salt, a suitable acid is added to the betaine solution. These acids can be organic or inorganic, monobasic or dibasic acids, with mineral acids being preferred.

Suitable acids include, e.g., HCl, HBr, HI, HF, $H_2NO_3$, $HClO_4$, HSCN, aliphatic mono-, di- or tricarboxylic acids, for example acetic acid, trifluoro acetic acid, trichloro acetic acid, or a preferred physiologically acceptable acid, such as, for example, the maleic acid, so as to provide a salt having the monomaleate anion HOOCCH=CHCOO- .Another suitable organic acid is naphthoic acid. Preferred cefquinome acid addition salts include cefquinome dihydrochloride, cefquinome dihydroiodide, cefquinome sulfate, cefquinome-6-hydroxy naphthoate, cefquinome-naphthoate, cefquinome 2,4 dihydroxy benzoate. The most preferred salt is the cefquinome sulfate.

Without wishing to be bound by theory, the inventors believe that the dosing scheme for the addition of the acid (e.g. sulfuric acid in the case of the preferred cefquinome sulfate) to the cefquinome betaine solution is of unexpected influence on the eventual particle size.

The dosing is done in a single shot. This does not exclude that the single shot could comprise a plurality of two or more overlapping shots, i.e. in which a first shot is followed by a second shot, and or further shots, without pause between the shots. However, this refers to a one-time shot by which all of the desired acid is added quickly in a very short time period (a very short time period (less than 15 min, preferably less than 10 min, especially 5 $\pm$ 2 minutes or in less than 5 min) in contrast to the pH adjustment to pH=1,3, as described in EP 280157 or to pH=1,8, as described in US 2006/0100424, which is a more time consuming procedure and takes more time to come to a constant pH.

The dosing is in a molar excess of at least 40% and less than 100%. This means that, as calculated on the amount of cefquinome present, the acid (i.e. sulfuric acid in the case of preparing cefquinome sulfate) is added in at least 1.4 equivalents and in less than 2.0 equivalents thereof. In view of processability, it is preferred that the excess is not as high. Preferably, particularly with a view to producing particles of a cefquinome acid addition salt, preferably cefquinome sulfate, satisfying the most preferred particle size distribution, the acid, preferably sulfuric acid, is added in a molar excess of 70-99% or 70- 90%, more preferably 75-85% and most preferably 80-85%. Thus, a single shot directly providing acid, preferably sulfuric acid, in a molar excess of 80-85% is the most preferred embodiment.

The aqueous betaine solution can be in any water miscible organic solvent like ketones or alcohols. Preferred solvents include acetone and ethyl alcohol, and most preferably a mixture is used of acetone and water. Herein the ratio of the two types of solvents (solvent/water) can vary in the range of 0 to 1.7, preferably 0.5 to 1.5, more preferably 1 to 1.5, and most preferably this solvent/water ratio is 1.2.

The use of mixed organic and aqueous solvents can be used with advantage in steering the process of particle precipitation (crystallization). Additionally the particle size distribution can be steered by conducting displacement washings of water.

The concentration of cefquinome betaine solution can vary from 0.165 to 0.196 mol/L. Preferably the cefquinome

is present in a concentration of 0.165 to 0.166 mol/L.

In order to produce particles, the addition of acid, preferably sulfuric acid, is followed by crystallization. It will be clear to the skilled person that, depending on the solvent and temperature, this crystallization will automatically occur (as is preferred) as a result of the formation of a sulfate upon the addition of acid, preferably sulfuric acid. This is particularly so when the preferred combination of organic and aqueous solvents, and most preferably acetone/water, is used.

With a view to obtaining particles having the preferred particle size, it is further preferred that the temperature upon crystallization be tailored. In general, from a processing point of view, acid, preferably sulfuric acid, can be added over a wide temperature range, from 0°C to 100 °C. However, it is preferred that the acid, preferably sulfuric acid, be added at a temperature (of the betaine solution), of 0°C - 35°C, preferably 15 °C -21 °C, and most preferably at a temperature of 19 °C-21 °C.

As a direct or indirect result of adding acid, preferably sulfuric acid, to the betaine solution, particles of cefquinome acid addition salt, preferably cefquinome sulfate, will precipitate. Reference is made herein to particles to reflect that these can be crystalline, amorphous, or a mixture of both. However, without wishing to be bound by theory, the inventors believe that the particles produced in accordance with the invention are in fact crystalline.

Surprisingly, by the method of the invention particles of cefquinome acid addition salts, preferably cefquinome sulfate particles, can be produced that differ from existing cefquinome acid addition salt particles, especially sulfate particles. The latter results in particles which must be adjusted to the desired particle size for good syringability and resuspendability of suspension injectables by micronization.

The invention method results in particles having a particle size distribution (PSD) comparable to the aforementioned PSD of milled or micronized material that have good syringability and resuspendability of suspension injectables (physical stability)

Surprisingly the particles produced in accordance with the invention comprise loose packed agglomerates of even smaller primary particles. These primary particles have particle sizes typically in the rage of 0.05 - 50 μm, and preferably 0.07 -10 μm, with at least 75% of the particles in the range of 0.07-0.3 μm and preferred in the range of 0.08-0.275 μm.

In connection herewith, the invention in one aspect includes novel particles of cefquinome acid addition salt, preferably cefquinome sulfate particles, obtainable by the foregoing method, which are agglomerates of primary particles of the aforementioned PSD range and which particularly are further characterized by the ability to reverse the agglomeration, e.g by simply agitating suspensions of the particles or under the influence of ultrasound.

Ultrasound is known to the skilled person. In the context of the invention, it is applied preferably using a sonotrode, also in combination with a flow cell and with cooling of the sonicated suspension e.g. employing a Bransson sonifier 250; Hielscher UIP1000; Sonorex Sonobloc. The particle size distribution can be steered by duration and energy input of the agitation or the sonication process.

With the particles of the invention, both agitation as well as sonication is believed to result in disintegrating of the agglomerates, without damaging the crystal lattice and/or the particle surface. This is fundamentally different from the existing particles, where milling (e.g. micronization) may lead to crystal lattice break-up, and the occurrence of highly irregular particle surfaces.

In this respect, the invention also provides particles of cefquinome acid addition salt, preferably cefquinome sulfate particles, of a size distribution $d(50) \leq 7$ μm, $d(90) \leq 15$ μm, and $d(100) \leq 50$ μm, wherein the particles are not micronized.

By providing the aforementioned novel particles, the invention opens up several favorable possibilities. One is that the formation of agglomerates enables tuning the process in such a way as to produce agglomerates with a desired size distribution within a wide range. It will be understood that the lower limit hereof is determined by the aforementioned size of the primary particles. The upper limit is generally of the order of a diameter of 1 mm. Preferably, the particle size varies from 1 μm to 500 μm, more preferably from 5 μm to 100 μm.

Particle sizes are determined by Laser light scattering, which is the method of choice to determine the particle size of particles between 0.1 and 3000 μm. This is more correctly called Low Angle Laser Light Scattering (LALLS). This method has become the preferred standard in many industries for characterization and quality control. The international standard ISO 13320-1 "Particle Size Analysis - Laser Diffraction Methods" describes the methods for the determination of particle size distribution via laser diffraction. The applicable range according to ISO13320 is 0.1 - 3000 μm. The method is conducted in accordance with Winnacker-Küchler: "Chemische Technologie", 4th Editionl.,volume 1, page. 46 ff.

The particle sizes, and the distribution thereof, can be influenced by the amount of excess of acid, preferably sulfuric acid. This enables the person skilled in the art seeking to produce particles of a cefquinome acid addition salt (preferably cefquinome sulfate particles), of a desired size, by varying the amount of excess of acid (preferably sulfuric acid), and doing a simple particle size measurement of the particles produced, to select the desired conditions for producing the particle sizes sought.

With reference to particle size distributions of the order of magnitude of the existing, micronized cefquinome, it is preferred that the molar excess of acid, preferably sulfuric acid, is 70-90%, more preferably 75-85% and most preferably 80-85%.

It should be noted that the novel particles of the invention exhibit further properties by which they are favorably distinct from existing cefquinome sulfate. For example the novel particles show different interactions with water, observable by Dynamic Vapor Sorption (DVS).

This determination relates to the water vapor theory, which is known to the skilled person. See Tisserand, C et al "Comparison of two techniques for the surface analysis of alumina: Inverse Gas Chromatography at Finite Concentration (IGC-FC) and Dynamic Vapor Sorption (DVS)" in Powder Technology 190, (2009) page 53-58 The novel particles of the invention show no tendency of hysteresis (Type II characteristic in the aforementioned theory) whereas the existing cefquinome sulfate particles show Type IV characteristic.

The particles of cefquinome acid addition salt, preferably cefquinome sulfate particles, of the present invention can be put to use in known manner, particularly in the form of a suspension in an oily base, e.g. ethyl oleate, MCT oil (see below) or, Miglyol® (see below). Herein the invention provides, as one of its advantages, suspensions of increased stability against sedimentation. Such stability against sedimentation can be measured with a macroscopic optical scanning device, TURBISCAN® (e.g. supplied by Formulaction, France), as described in WO 01/17504). The TURBISCAN® equipment detects any change (e.g., clarification, sedimentation, etc.) in dispersed systems on the basis of multiple light scattering. It is a vertical scan macroscopic analyser consisting of a reading head moving along a flat-bottomed cylindrical cell, while scanning the entire sample height (see e.g. Mengual, O, " Characterisation of instability of concentrated dispersions by a new optical analyser: the TURBISCAN MA 100", Colloids and Surfaces A Physicochemical and Engineering Aspects 152 (1999), page 111-123..

The base preferably comprises a pharmaceutical acceptable low viscosity oily medium, such as medium chain triglyceride or a mixture of medium chain triglycerides. Medium chain triglycerides (MCT oil) have fatty acid chains of 6 - 12 carbon atoms and for the medically refined grades of MCT oil each chain has 8 - 10 carbon atoms. The MCT oil may comprise either triglycerides of the C8-C10 fatty acids, or propylene glycol diesters of these fatty acids or a mixture of both triglycerides and propylene glycol diesters. Preferably these C8 -C10 fatty acids are fully saturated, such as n-caprylic and n-capric acids. These are conveniently prepared by the commercial fractionating of naturally occurring vegetable (e.g. coconut) oil to give mainly C8-10 fatty acids followed by esterification of these acids with a chosen alcohol. Fractionated vegetable oil having the desired composition is commercially available. Proprietary examples of such oils are Miglyol® 812 as capric/caprylic triglycerides and Miglyol® 840 as propylene glycol dicaprylate/caprate.

Equivalents of these oils are for example: Aldo® MCT KFG, Ado® TC, Calgene CC-33, Calgene CC-33-F, Calgene CC-33-L, Calgene CC-33-S, Captex(R) 300, Captex® 355, Crodamol GTCC, Estasan GT 8-40 3578, Estasan GT 8-60 3575, Estasan GT 8-60 3580, Estasan GT 8-65 3577, Estasan GT 8-65 3581 , Estasan GT 8-70 3579, Labrafac® LIPO, Labrafac® lipophile WL 1349, Lexol® GT-855, Lexol® GT-865, Miglyol® 810, Miglyol® 812, Myritol® 312, Myritol® 318, Neobee® 1053, Neobee® M-5, Neobee® O, Pelemol® CCT, Standamul® 318, Standamul® 7105 and Calgene CC-22, Calgene CC-22-S, Captex® 200, Lexol® PG-865, Miglyol® 840, Myritol® PC, Neobee® 1054, Neobee® M-20, Pelemol® PDD, Standamul® 302.

Most preferred is Miglyol® grade 812. The composition according to the invention comprises a thickener. A thickener in a pharmaceutical formulation in general is useful to provide good suspending properties and increases the viscosity of the composition without negatively affecting the syringability.

It will be understood that the present invention does not necessarily change any aspects of the process other than the step of adding acid, preferably sulfuric acid, to the cefquinome betaine solution, and the formation of particles thereupon.

The cefquinome particles of the invention can be put to use in at least the same ways as the existing cefquinome. The term "cefquinome" when used herein includes pharmaceutical acceptable salts and esters thereof.

Cefquinome (INN-International Non-proprietary Name) is the first fourth-generation cephalosporin developed for use in veterinary medicine. It is a semisynthetic aminothiazolyl cephalosporin resembling cefotaxime, but with a bicyclic pyridinium group at the C-3 position (Isert et al, Seibert et al, 29th Interscience Conference on Antimicrobial Agents and Chemotherapy Houston, Texas, 1989).

Cefquinome has been found to be especially useful in treatment of respiratory infections in livestock (e. g. cattle and pigs (particularly Mannheimia haemolytica infection in cattle) when administered by injection.

Various crystalline cephalosporin salts have been proposed for the treatment of bacterial infections, e. g. cefquinome dihydrochloride or cefquinome sulfate or crystalline cephalosporin addition salts with particularly low solubility e. g. cefquinome-6 hydroxynaphthoate (cefquinome-naphthoate) and cefquinome 2,4 dihydroxy benzoate (cefquinome hydroxy benzoate). Preferred is cefquinome sulfate.

The cefquinome particles of the present invention will be generally incorporated into a pharmaceutical composition, preferably a suspension as described hereinbefore. A typical pharmaceutical composition according to the invention comprises 2.0 to 20.0 % by weight of cefquinome. The composition according to the invention can be applied to an

animal in general by all application forms known in the art. Generally the administration to the animal is done orally or parenterally. While the pharmaceutical composition according to the current invention is preferably administered parenterally, e. g. by intramuscular or subcutaneous injection, treatment via alternative routes is also possible.

In general the composition according to the current invention can be administered to all species of animals that need treatment or prevention of bacterial infections such as pigs, cattle, horses, goats, sheep, cats, dogs, poultry and fish.

Specific diseases that can be are bacterial infections of respiratory tract, urogenital tract, soft tissue-and skin infections and mastitis or metritis.

The particular amount of cefquinome required for a particular treatment will vary, depending upon the species, age and weight of the host animal being treated, the particular disease to be guarded against, or treated, as well as the specific antimicrobial agent selected for the treatment, the route and the frequency of administration. For example the dose of cefquinome sulfate (or other acid addition salt) for the treatment of horses, sheep, goat, poultry and fish is between 5 to 10 mg/kg bodyweight. For cattle a dosage of 5 mg/kg bodyweight is recommended and for the application to pig, dog and cat a dosage of 10 mg/kg bodyweight.

Preferred uses are in the following treatments:

Cattle: Respiratory disease caused by *Pasteurella multocida and Mannheimia haemolytica*; Digital dermatitis, infectious bulbar necrosis and acute interdigital necrobacillosis (foul in the foot); Acute *E.coli* mastitis with signs of systemic involvement. Calves: E.coli septicaemia in calves. Pigs: For the treatment of bacterial infections of the lungs and respiratory tract caused by *Pasteurella multocida, Haemophilus parasuis, Actinobacillus pleuropneumoniae, Streptococcus suis* and other cefquinome-sensitive organisms; Mastitis-Metritis-Agalactia syndrome (MMA) with involvement of *E.coli, Staphylococcus spp., Streptococcus spp.* and other cefquinome sensitive organisms; Piglets: Reduction of mortality in cases of meningitis caused by Streptococcus suis. Another preferred use is in the treatment of Arthritis caused by *Streptococcus ssp., E. coli* and other cefquinome-sensitive organisms and Epidermitis (mild or moderate lesions) caused by *Staphylococcus hyicus.*

In summary, the invention includes a process for the production of particles of cefquinome acid addition salt, preferably cefquinome sulfate particles, by precipitation of cefquinome salt from a cefquinome betaine solution, wherein acid, preferably sulfuric acid, is added to the betaine solution. According to an aspect of the invention the acid, preferably sulfuric acid, is added in a single shot in a very short time (less than 15 minutes), in a molar excess of 40% to less than 100%. As a result, particles are formed that comprise agglomerates of microscale primary crystalline particles. This enables providing cefquinome acid addition salt, preferably cefquinome sulfate, in particle sizes commensurate with milled, especially micronized material, but with an improved physical stability as compared therewith.

The invention will now be further described with reference to the following, non-limiting, examples, and the accompanying figures.

**Preparation of cefquinome particles: Examples 1 - 7**

**Example 1:** 250.3 g of Cefquinome sulfate were suspended at a temperature below 10°C in 740 ml of water before adding 150 ml of 16.3% NaOH, followed by 1020 ml of acetone for precipitation of sodium sulfate ($Na_2SO_4$) at -5°C. After filtration and washing of the salt with 375 ml of a mixture of acetone/water = 1.27/1 the combined liquids were decolourized by treatment with 21 g of charcoal. After washing of the charcoal with 150 ml of acetone/water = 2/1, the mixture of the combined liquids was heated to 19°C before addition of 388 ml of 15.1% sulphuric acid within 5 minutes. Stirring of the reaction mixture was continued at 19-21 °C for 35 min before addition of 1200 ml of acetone. Under stirring the suspension was poured into 12400 ml of acetone. After filtration and washing twice with 2500 ml of acetone the precipitate was dried over night at 32°C to give 225 g of the material used for preparation of ethyl oleate formulations A, B or C. Samples of the material for Particle Size Distribution (PSD) were taken after the addition of the sulphuric acid directly before addition of acetone. Ultrasound was applied for 60 seconds.

PSD: D(100) = 15.14 μm; D(90) = 6.25 μm; D(50) = 3.05.

**Example 2:)** Comparable to the procedure as described in example 1 1200 ml of ^water and 500 g of Cefquinome sulfate were reacted with 350 g of 16.3% NaOH and 1325 ml of acetone at 5°C. The salt and the 15 g of charcoal both were washed with 300 ml of acetone/water = 3/1 each, followed by 215 ml of acetone/water = 3,3/1 for additional charcoal washing, resulting in 3472 g of the combined liquids, which were used as stock solution. This stock solution was divided into three equal portions, to be used in Examples 2-1, 2-2 and 2-3.

**Example 2-1:** One portion of 1158 g of the stock solution was heated to 18°C. 334 ml of acetone were added before dosage of 286 g of 15.1% sulphuric acid within 5 minutes. The reaction mixture was stirred and then diluted with 800 ml of acetone under continues stirring. Samples of the material for Particle Size Distribution (PSD) were taken after the addition of the sulphuric acid directly before addition of acetone. Ultrasound was applied for 60 seconds.
PSD: D(100) = 104.71 $\mu$m; D(90) = 36.24 $\mu$m; D(50) = 9.67 $\mu$m

**Example 2-2:** To another portion of 1159 g of the stock solution 95 ml of acetone were added and the procedure as described for example 2-1 was followed. 605 ml of the suspension were diluted with 1800 ml of acetone and the precipitate was separated and dried at 30°C under reduced pressure. Samples of the material for Particle Size Distribution (PSD) were taken after the addition of the sulphuric acid directly before addition of acetone. Ultrasound was applied for 60 seconds.
PSD: D(100) = 15.14 $\mu$m; D(90) = 4.86 $\mu$m; D(50) = 2.58 $\mu$m

**Example 2-3** To an other portion of 1155 g of the stock solution additional 140 ml of acetone were added compared to the volume as described in example 2-2 and 117 ml of water before the dosage of sulphuric acid as described in example 2-1 was performed at 18°C. 670 ml of the suspension were treated with 2000 ml of acetone and the precipitate was separated and dried at 30°C under reduced pressure. Samples of the material for Particle Size Distribution (PSD) were taken after the addition of the sulphuric acid directly before addition of acetone. Ultrasound was applied for 60 seconds.
PSD: D(100) = 22.91 $\mu$m; D(90) = 6.95 $\mu$m; D(50) = 2.98 $\mu$m

**Example 3:)** A stock solution was prepared according to example 2 (3505 g). This stock solution was divided into three equal portions.

**Example 3-1** Comparable to the procedure as described in example 2-1 a portion of 1166 g of the stock solution 3 was heated to 29°C before following the procedure as described for example 2-1. Before addition of 800 ml of acetone as described in example 2-1 the suspension was cooled to 3 °C. At a temperature of 10°C 700 ml of acetone were added to 266 ml of this suspension and the precipitate separated and dried. Samples of the material for Particle Size Distribution (PSD) were taken after the addition of the sulphuric acid directly before addition of acetone. Ultrasound was applied for 60 seconds.
PSD: D(100) = 15.14 $\mu$m; D(90) = 5.97 $\mu$m; D(50) = 2.92 $\mu$m

**Example 3-2** The procedure as described in example 3-1 was followed with 1170 g of the stock solution 3 and 95 ml of acetone added before the addition of the acid within 5 minutes. Before separation and drying of the precipitate 725 ml of acetone were added to 240 ml of the suspension. Samples of the material for Particle Size Distribution (PSD) were taken after the addition of the sulphuric acid directly before addition of acetone. Ultrasound was applied for 60 seconds.
PSD: D(100) = 13.18 $\mu$m; D(90) = 5.55 $\mu$m; D(50) = 2.87 $\mu$m

**Example 3-3** The procedure as described in example 3-2 was followed with a portion of 1169 g of the stock solution 3 and with additional 139 ml of acetone and 117 ml of water added before the addition of the acid within 5 minutes. Before separation and drying of the precipitate 820 ml of acetone were added to 260 ml of the suspension. Samples of the material for Particle Size Distribution (PSD) were taken after the addition of the sulphuric acid directly before addition of acetone. Ultrasound was applied for 60 seconds.
PSD: D(100) = 13.18 $\mu$m; D(90) = 5.78 $\mu$m; D(50) = 2.96 $\mu$m

Examples 4-7 are to demonstrate the influence of sulphuric acid variation on the PSD

**Example 4:** With exception of the charcoal treatment the procedure as described in example 1 was followed in general for reacting 50 g of Cefquinome sulfate with 32 g of 15% NaOH and subsequent precipitation of the salt at a temperature below 10°C. Cefquinome sulfate was precipitated using 66 ml of 15% sulphuric acid added within 1-2 minutes. After work up 45.7 g of dry material were isolated. Samples of the material for Particle Size Distribution (PSD) were taken after the addition of the sulphuric acid directly before addition of acetone. Ultrasound was applied for 60 seconds.
PSD: D(100) = 60.26 $\mu$m; D(90) = 35.40 $\mu$m; D(50) = 18.91 $\mu$m

**Example 5** With 70 ml of 15% sulphuric acid, added within 1-2 minutes, 45.8 g of dry material were isolated when

following the procedure as described in example 4. Samples of the material for Particle Size Distribution (PSD) were taken after the addition of the sulphuric acid directly before addition of acetone. Ultrasound was applied for 60 seconds.
PSD: D(100) = 30.20 μm; D(90) = 14.72 μm; D(50) = 6.56 μm

**Example 6:** With 75 ml of 15% sulphuric acid added within 1-2 minutes, 44.8 g of dry material were isolated when following the procedure as described in example 4. Samples of the material for Particle Size Distribution (PSD) were taken after the addition of the sulphuric acid directly before addition of acetone. Ultrasound was applied for 60 seconds.
PSD: D(100) = 13.18 μm; D(90) = 6.04 μm; D(50) = 3.05 μm

**Example 7:** With 84 ml of 15% sulphuric acid added within 1-2 minutes, 45 g of dry material were isolated when following the procedure as described in example 4. Samples of the material for Particle Size Distribution (PSD) were taken after the addition of the sulphuric acid directly before addition of acetone. Ultrasound was applied for 60 seconds.
PSD: D(100) = 15.14 μm; D(90) = 7.05 μm; D(50) = 3.71 μm

Example 8 The process of Example 6 including the charcoal treatment was scaled-up by a factor of 10. Samples of the material for Particle Size Distribution (PSD) were taken after the addition of the sulfuric acid within 5 minutes directly before addition of acetone. PSD was measured after 1 minute and after 10 minutes of only stirring in the measurement equipment.
PSD (1 minute stirring): D(100)=239,9μm; DE(90)=66,97μm; D(50)=4,78μm;
PSD (10 minutes stirring): D(100)=11,48μm; DE(90)=5,95μm; D(50)=3,34μm;
Applying additional 5-6 minutes of ultra sound the PSD is as follows: PSD (10 minutes stirring + 6 minutes of Ultrasound): D(100)=5,01μm; DE(90)=1,98μm; D(50)=0,15μm;

**Preparation of ethyloleate formulations**

Formulations A - C with cefquinome sulfate particles of Example 1 were prepared as following:

Formulation A

A 2 L beaker was positioned in an ice bath. For a calculated batch size of 1200 ml 1027 g of ethyl oleate were weighed into the beaker and 35.7 g of dry cefquinome sulfate material as produced in example 1 were added. The suspension was homogenized using an IKA Ultraturrax Typ 50 basic with IKA dispersing stirrer S50N-G45F for 120 minutes with a stirrer speed of 10000 rpm. The temperature was kept below 25°C. 962 g of the suspension were isolated.

Formulation B

A 2 L beaker was positioned in an ice bath. For a calculated batch size of 1200 ml 1027 g of ethyl oleate were weighed into the beaker and 35.7 g of dry cefquinome sulfate material as produced in example 1 were added. The suspension was homogenized using an IKA Ultraturrax Typ 50 basic with IKA dispersing stirrer S50N-G45F for 165 minutes with a stirrer speed of 10000 rpm. For disintegration of the agglomerates with ultrasound a Branson Sonifier 250 with a 6.5 mm ultrasound sonotrode as converter was used. The ultrasound energy input was adjusted to ~ 100 Watt. The temperature was kept below 31 °C by spacing the sonication and cooling down the suspension to ~ 10°C before further sonication. 992 g of the suspension were isolated.

Formulation C

An ethyl oleate suspension comparable to Formulation B was prepared by adding a suspension of the corresponding quantity of material obtained from example 1 in a mixture of acetone and 2-propanole, which was homogenized with 5200 rpm and sonicated as for Formulation B for 35 minutes to the calculated quantity of ethyl oleate. The mixture was stirred for another 50 minutes. The low boiling suspension liquids were removed at 35°C under reduced pressure until constant weight.

The particle size was determined with a Malvern Master Sizer GMAL 01 with the Hydro 2000G measuring cell according to Frauenhofer method.

Table 1: Particle Size Distribution of Formulations A-C and prior art micronized 2.5% cefquinome sulphate suspension

| | **D(0.50) μm** | **D(0.90) μm** | **D(100) μm** |
|---|---|---|---|
| prior art non micronized material | 63,01 | 114,75 | 178,25 |
| prior art micronized 2.5% cefquinome sulphate suspension | 5.14 | 14.89 | 34.67 |
| Formulation A | 5.63 | 13.60 | 34.67 |
| Formulation B | 2.82 | 6.16 | 17.38 |
| Formulation C | 3.60 | 6.70 | 13.18 |

Result: All tested formulations meet the beneficial particle size distribution of d(50) ≤ 7 μm, d(90) ≤ 15 μm, and d(100) ≤ 50 μm.

**Physical stability of Formulation A-C**

Figure 1- 4 represent the clarification kinetic of Formulations A-C and Cobactan 2.5% determined by a macroscopic optical scanning device, TURBISCAN® (supplied by Formulaction, France), in the middle of the measuring cell for 4 hours.

The TURBISCAN® equipment detects any change (e.g., clarification, sedimentation, etc.) in dispersed systems on the basis of multiple light scattering. It is a vertical scan macroscopic analyser consisting of a reading head moving along a flat-bottomed cylindrical cell, while scanning the entire sample height. The reading head itself consists of a pulsed near infrared light source and two synchronous detectors: the transmission detector picks up the light transmitted through the product and the backscattering detector receives the light backscattered by the product. The reading head acquires transmission and backscattering data every 40 μm on a maximum height of 80 mm. The profile obtained characterises the product homogeneity, particles concentration and mean diameter. Results are represented by the percentage of backscattered or transmitted light as a function of the sample height (in mm). The acquisition along the product is then repeated with a programmable frequency to obtain a superimposition of product fingerprints characterising the stability or instability of the product, whether they are identical or not.

Results. No sign of physical instability (no clarification, no sedimentation in the middle of the cell) within 4 hours was measured by TURBISCAN® for the ethyl oleate formulations A-C that were manufactured with cefquinome sulfate particles from Example 1 (see Figure 1-4).

## Claims

1. A process for the production of particles of a cefquinome acid addition salt by precipitation of the cefquinome acid addition salt from a cefquinome betaine solution, wherein acid is added to the betaine solution, and wherein the addition of the acid is done in a single shot in a molar excess of 40% to less than 100%.

2. A process according to claim 1, wherein the acid is a mineral acid.

3. A process according to claim 2, wherein the mineral acid is sulfuric acid.

4. A process according to claim 1 to 3, wherein the molar excess is 70 - 90%.

5. A process according to claim 4, wherein the molar excess is 80 - 85%.

6. A process according to any one of the preceding claims, wherein the acid is added at a temperature of the betaine solution, of 0°C to 35°C, preferably 15°C to 21°C.

7. Particles of a cefquinome acid addition salt, preferably cefquinome sulfate particles, obtainable by a method according to any one of the preceding claims.

8. Particles of a cefquinome acid addition salt according to claim 7, preferably of cefquinome sulfate, consisting essentially of primary crystalline particles of which more than 75% has a particle size in the range of from 0,08 μm to 0,275 μm.

9. A crystalline cefquinome salt according to claim 7, wherein the primary particles form agglomerates of an agglomerated particles size ranging from 1 μm to 500 μm, preferably from 5 μm to 100 μm.

10. A pharmaceutical formulation comprising cefquinome acid addition salt particles, preferably cefquinome sulfate particles, according to any one of the claims 7 to 9, and a pharmaceutically acceptable carrier.

11. A pharmaceutical formulation according to claim 10, wherein the formulation comprises a suspension of the cefquinome particles in an oily medium, preferably selected from the group consisting of ethyl oleate, and medium chain triglycerides.

12. A pharmaceutical formulation according to claim 10 or 11 for use in the treatment or prevention of bacterial infections in animals such as pigs, cattle, horses, goats, sheep, cats, dogs, poultry and fish.

## Patentansprüche

1. Verfahren zur Herstellung von Teilchen eines Cefquinom-Säureadditionssalzes durch Ausfällen des Cefquinom-Säureadditionssalzes aus einer Cefquinom-Betainlösung, wobei die Betainlösung mit Säure versetzt wird und wobei das Versetzen mit der Säure auf einmal mit einem molaren Überschuss von 40% bis weniger als 100% erfolgt.

2. Verfahren nach Anspruch 1, wobei es sich bei der Säure um eine Mineralsäure handelt.

3. Verfahren nach Anspruch 2, wobei es sich bei der Mineralsäure um Schwefelsäure handelt.

4. Verfahren nach Anspruch 1 bis 3, wobei der molare Überschuss 70-90% beträgt.

5. Verfahren nach Anspruch 4, wobei der molare Überschuss 80-85% beträgt.

6. Verfahren nach einem der vorhergehenden Ansprüche, wobei die Säure bei einer Temperatur der Betainlösung von 0°C bis 35°C, vorzugsweise 15°C bis 21°C, zugesetzt wird.

7. Teilchen eines Cefquinom-Säureadditionssalzes, vorzugsweise Cefquinom-Sulfat-Teilchen, die nach einem Verfahren nach einem der vorhergehenden Ansprüche erhältlich sind.

8. Teilchen eines Cefquinom-Säureadditionssalzes nach Anspruch 7, vorzugsweise von Cefquinom-Sulfat, die im Wesentlichen aus kristallinen Primärteilchen bestehen, von denen mehr als 75% eine Teilchengröße im Bereich von 0,08 μm bis 0,275 μm aufweisen.

9. Kristallines Cefquinom-Salz nach Anspruch 7, wobei die Primärteilchen Agglomerate mit einer agglomerierten Teilchengröße im Bereich von 1 μm bis 500 μm, vorzugsweise 5 μm bis 100 μm, bilden.

10. Pharmazeutische Formulierung, die Cefquinom-Säureadditionssalz-Teilchen, vorzugweise Cefquinom-Sulfat-Teilchen, nach einem der Ansprüche 7 bis 9 sowie einen pharmazeutisch unbedenklichen Träger umfasst.

11. Pharmazeutische Formulierung nach Anspruch 10, wobei die Formulierung eine Suspension der Cefquinom-Teilchen in einem öligen Medium, vorzugsweise ausgewählt aus der Gruppe bestehend aus Ethyloleat und mittelkettigen Triglyceriden, umfasst.

12. Pharmazeutische Formulierung nach Anspruch 10 oder 11 zur Verwendung in der Behandlung oder Vorbeugung von bakteriellen Infektionen bei Tieren wie Schweinen, Rindern, Pferden, Ziegen, Schafen, Katzen, Hunden, Geflügel und Fischen.

## Revendications

1. Procédé de production de particules d'un sel d'addition d'acide de cefquinome par précipitation du sel d'addition d'acide de cefquinome dans une solution de cefquinome bétaïne, de l'acide étant ajouté à la solution de bétaïne, et l'addition de l'acide étant effectuée en une seule fois dans un excès molaire de 40 % à moins de 100 %.

2. Procédé selon la revendication 1, **caractérisé en ce que** l'acide est un acide minéral.

3. Procédé selon la revendication 2, **caractérisé en ce que** l'acide minéral est l'acide sulfurique.

4. Procédé selon les revendications 1 à 3, **caractérisé en ce que** l'excès molaire est de 70-90 %.

5. Procédé selon la revendication 4, **caractérisé en ce que** l'excès molaire est de 80-85 %.

6. Procédé selon l'une quelconque des revendications précédentes, **caractérisé en ce que** l'acide est ajouté à une température de la solution de bétaïne de 0°C à 35°C, de préférence de 15°C à 21°C.

7. Particules d'un sel d'addition d'acide de cefquinome, de préférence particules de sulfate de cefquinome, susceptibles d'être obtenues par une méthode selon l'une quelconque des revendications précédentes.

8. Particules d'un sel d'addition d'acide de cefquinome selon la revendication 7, de préférence de sulfate de cefquinome, constituées essentiellement de particules cristallines primaires dont plus de 75 % ont une taille de particules dans la gamme de 0,08 μm à 0,275 μm.

9. Sel de cefquinome cristallin selon la revendication 7, **caractérisé en ce que** les particules primaires forment des agglomérats d'une taille de particules agglomérées allant de 1 μm à 500 μm, de préférence de 5 μm à 100 μm.

10. Formulation pharmaceutique comprenant des particules de sel d'addition d'acide de cefquinome, de préférence des particules de sulfate de cefquinome, selon l'une quelconque des revendications 7 à 9, et un support pharmaceutiquement acceptable.

11. Formulation pharmaceutique selon la revendication 10, **caractérisée en ce que** la formulation comprend une suspension des particules de cefquinome dans un milieu huileux, de préférence choisi dans le groupe constitué par l'oléate d'éthyle, et des triglycérides à chaîne moyenne.

12. Formulation pharmaceutique selon la revendication 10 ou 11, destinée à être utilisée dans le traitement ou la prévention d'infections bactériennes chez des animaux tels que le cochon, le bovin, le cheval, la chèvre, le mouton, le chat, le chien, la volaille et le poisson.

Figure 1: Sedimentation (Turbiscan 4 hours). Formulation A

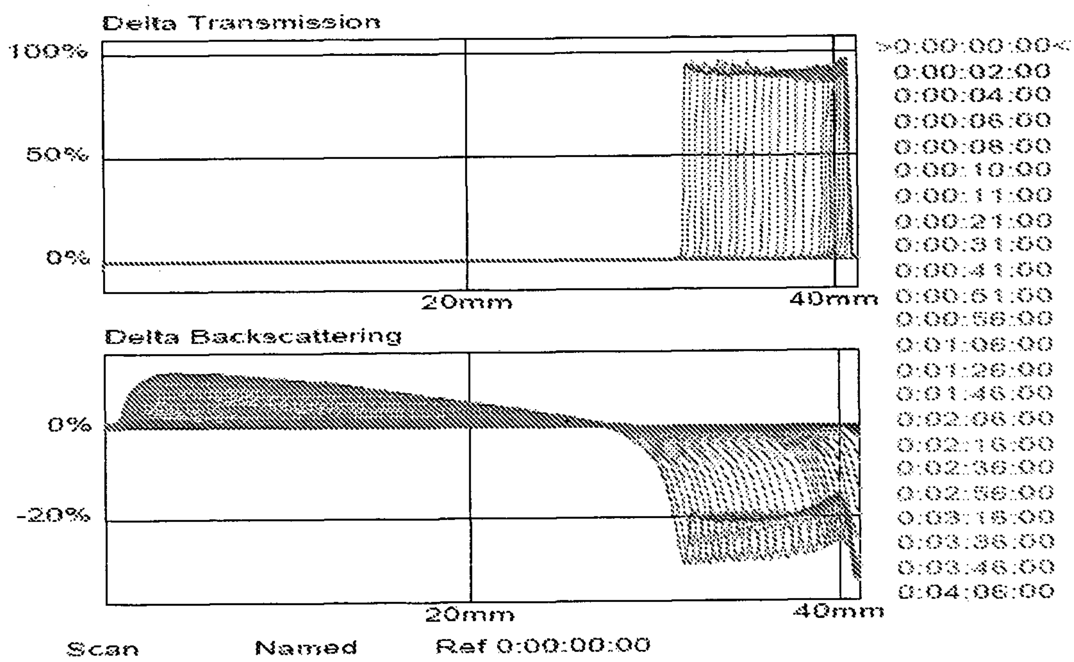


Figure 2 : Sedimentation ( Turbiscan 4 hours) Formulation B

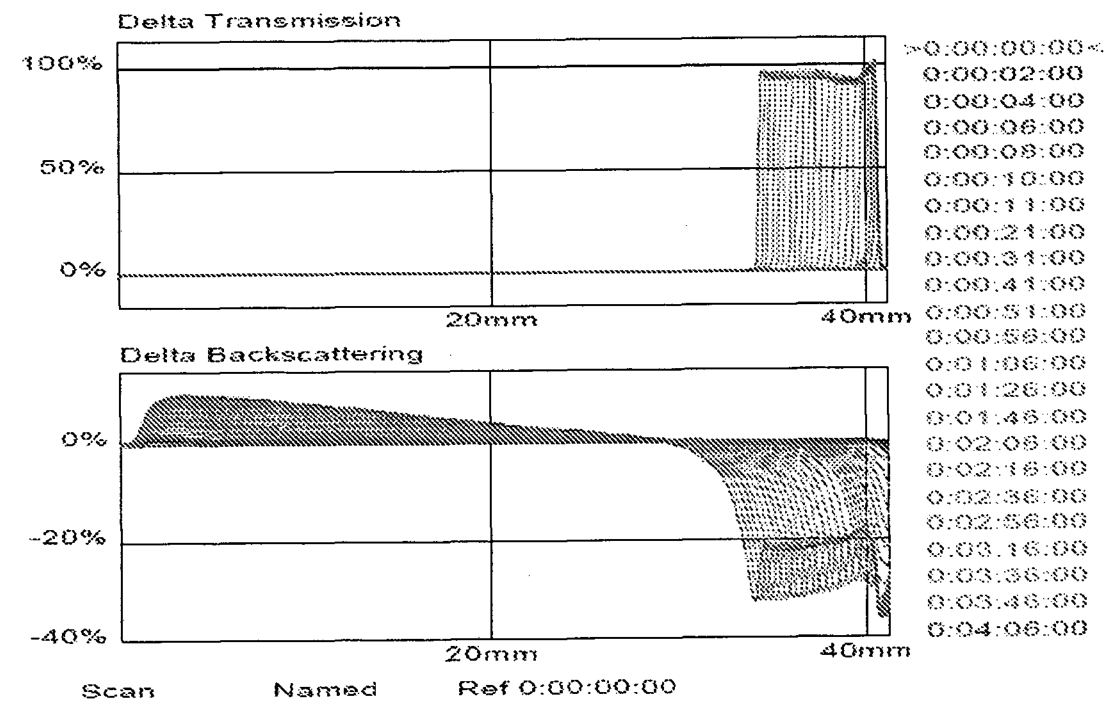

Figure 3 : Sedimentation (Turbiscan 4 hours) Formulation C

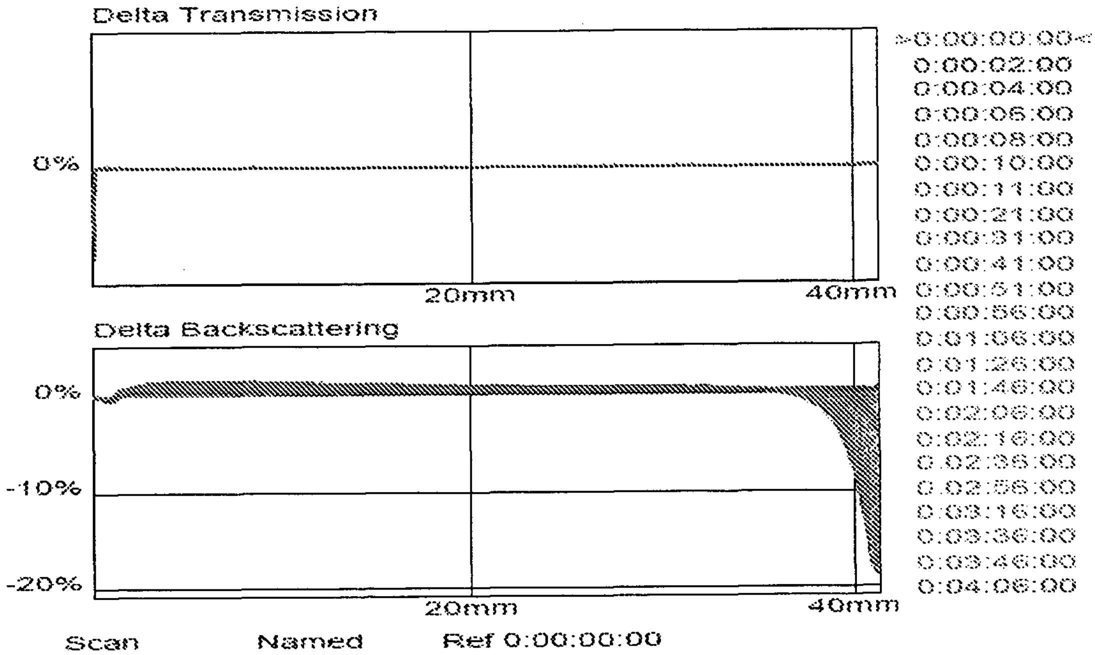


Figure 4 :Sedimentation (Turbiscan4 hours ) prior art micronized 2.5% cefquinome sulphate suspension

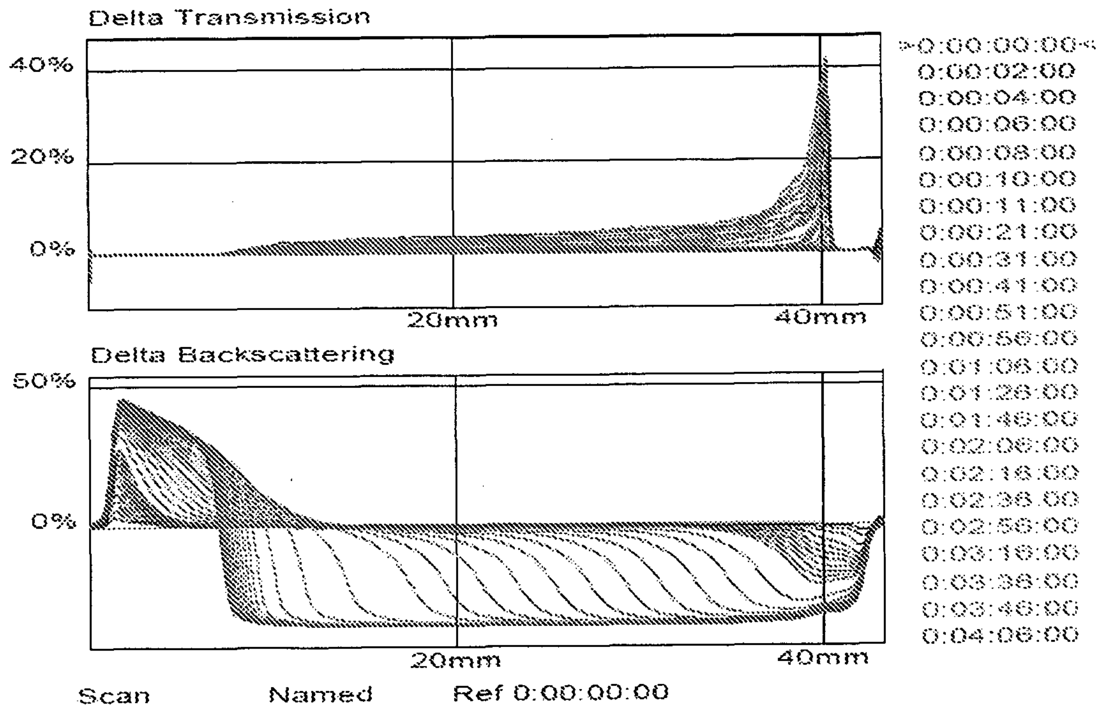

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- EP 280157 A
- US 20060100424 A
- WO 03063877 A
- WO 0117504 A


### Non-patent literature cited in the description


- **TISSERAND, C et al.** Comparison of two techniques for the surface analysis of alumina: Inverse Gas Chromatography at Finite Concentration (IGC-FC) and Dynamic Vapor Sorption (DVS. *Powder Technology,* 2009, vol. 190, 53-58
- **MENGUAL, O.** Characterisation of instability of concentrated dispersions by a new optical analyser: the TURBISCAN MA 100. *Colloids and Surfaces A Physicochemical and Engineering Aspects,* 1999, vol. 152, 111-123
- **ISERT ; SEIBERT et al.** *29th Interscience Conference on Antimicrobial Agents and Chemotherapy Houston, Texas,* 1989